# EUROPÄISCHE PATENTSCHRIFT

(11) **EP 3 350 153 B1**
(45) Veröffentlichungstag und Bekanntmachung des Hinweises auf die Patenterteilung: **05.06.2019**
(21) Anmeldenummer: 16763864.2
(22) Anmeldetag: 14.09.2016
(51) Int. Cl.: C07C 67/39, C07C 67/44

(54) **EXTRAKTIVE AUFARBEITUNG EINES NATRIUMSALZHALTIGEN MMA-METHANOL GEMISCHES**
EXTRACTIVE PROCESSING OF A SODIUM SALINE MMA METHANOL MIXTURE
PREPARATION EXTRACTIVE D'UN MELANGE DE METHANOL MMA CONTENANT DU SEL DE SODIUM

(30) Priorität: 16.09.2015 EP 15185434
(43) Veröffentlichungstag der Anmeldung: 25.07.2018
(73) Patentinhaber: Evonik Röhm GmbH, 64293 Darmstadt (DE)
(72) Erfinder: GRÖMPING, Matthias, 64295 Darmstadt (DE); KRILL, Steffen, 64367 Mühltal (DE); LYGIN, Alexander, 64347 Griesheim (DE); LUKIC, Milica, 60438 Frankfurt (DE); FINGER, Steffen, 65239 Hochheim am Main (DE)
(74) Vertreter: Evonik Patent Association
(86) Internationale Anmeldenummer: PCT/EP2016/071608
(87) Internationale Veröffentlichungsnummer: WO 2017/046110

(56) Entgegenhaltungen:
- WO-A1-2014/170223

## Beschreibung

### Gebiet der Erfindung

Die vorliegende Erfindung betrifft ein Verfahren zur Herstellung von Methylmethacrylat durch direkte oxidative Veresterung von Methacrolein. Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff.

Insbesondere betrifft die vorliegende Erfindung eine optimierte Aufarbeitung des Reaktoraustrags der oxidativen Veresterung von Methacrolein, mittels der Prozessabfallströme minimiert werden können und Prozesswasser auf optimale Weise recycliert werden kann. Außerdem wird die im Prozess anfallende Methacrylsäure optimal zurückgewonnen und dabei isoliert oder zum Wertstoff Alkylmethacrylat umgesetzt. Darüber hinaus hat dieses Verfahren den Vorteil, dass an die Apparative Ausgestaltung der Anlage weniger Ansprüche als im Stand der Technik beschrieben gestellt werden müssen.

### Stand der Technik

Methylmethacrylat (MMA) wird heute mittels diverser Verfahren, die von C₂- C₃- oder C₄-Bausteinen ausgehen, hergestellt. In einem dieser Verfahren wird MMA durch Oxidation von Isobutylen oder tert-Butanol mit Luftsauerstoff in der Gasphase am heterogenen Kontakt zu Methacrolein und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben. Nachteil dieses Verfahrens ist insbesondere ein sehr hoher Energiebedarf. In einer Weiterentwicklung des Verfahrens wird das Methacrolein in der ersten Stufe aus Propanal und Formaldehyd gewonnen. Ein solches Verfahren ist in WO 2014/170223 beschrieben.

In US 5,969,178 ist ein solches Verfahren zur oxidativen Umsetzung von Isobuten oder tert-Butanol zu Methacrolein und die folgende oxidative Veresterung zu MMA beschrieben. In dieser zweiten Stufe wird eine flüssige, im Wassergehalt reduzierte Mischung aus Methacrolein und Methanol mit molekularem Sauerstoff und einem Palladiumkatalysator umgesetzt, wobei dieser zumeist auf einem Träger als Palladium- Blei-Katalysator vorliegt. Aus dem Rohprodukt der oxidativen Veresterung wird darauf in einer ersten Destillationsstufe ein Gemisch aus Methacrolein und Methanol unterhalb des Kopfes der Kolonne abgetrennt, während über Kopf leichtsiedende Bestandteile entfernt werden. Der MMA-haltige Sumpf wird darauf in eine zweite Destillationsstufe geführt, in der über Kopf ein Azeotrop aus Methanol und gesättigten Kohlenwasserstoffen abgetrennt wird. Der Sumpf, enthaltend das Roh-MMA wird einer weiteren Aufarbeitung zugeführt, während aus der über Kopf gewonnen Fraktion mittels einem Phasentrenner und einer dritten Destillationskolonne Methanol isoliert und zurück in den Reaktor geführt wird. Dabei ist zu berücksichtigen, dass das Methanol aufgrund des gebildeten Azeotrops relativ viel Wasser enthalten kann und damit einer Entwässerung zugeführt werden muss.

Als Alternative zu diesem Verfahren offenbart die US 5,969,178 die Aufarbeitung in nur einer Kolonne, wobei sich bei dieser der Zulauf zwingend oberhalb des Sumpfes befindet. Über Kopf werden dieser Kolonne leichtsiedende Bestandteile aus dem Reaktoraustrag entfernt. Im Sumpf verbleibt eine Mischung aus Roh-MMA und Wasser, welche einer weiteren Aufarbeitung zuzuführen ist. Über einen Seitenstrom, dessen genaue Lage zunächst bestimmt werden muss und die durch Hinzufügen verschiedener Siedeböden eingestellt werden kann, wird der Kolonne schließlich eine Mischung aus Methacrolein und Methanol, gedacht zur Zurückführung in den Reaktor, entnommen. US 5,969,178 weist dabei selbst darauf hin, dass ein solches Verfahren aufgrund diverser Atzeotrope schwierig durchzuführen ist. Darüber hinaus spielt dabei insbesondere Methacrylsäure, die immer als Nebenprodukt vorliegt eine große Rolle. Nach diesem Verfahren, auch wenn die US 5,969,178 darüber schweigt, würde die Methacrylsäure derart abgetrennt werden, dass sie in einer der Entsorgung zuzuführenden Phase verbleibt und sich eine Isolierung nur bedingt lohnen würde. Damit jedoch sinkt die gesamtausbeute an methacrylischen Produkten dieses Verfahrens.

In US 7,012,039 wird eine etwas abweichende Aufarbeitung des Reaktoraustrags der oxidativen Veresterung offenbart. Hier wird in einer ersten Destillationsstufe über Siebböden Methacrolein über Kopf abdestilliert und die wässrige, MMA haltige Mischung aus dem Sumpf in
Einen Phasenseperator geleitet. In diesem wird die Mischung durch Zugabe von Schwefelsäure auf einen pH-Wert von ca. 2 eingestellt. Die Trennung des schwefelsauren Wassers von der organsichen bzw. ÖlPhase erfolgt dann mittels Zentrifugieren. Diese Ölphase wird in einer weiteren Destillation in hochsiedende Bestandteile und eine MMA haltige Phase, welche über Kopf abgezogen wird, getrennt. Die MMA haltige Phase wird danach in einer dritten Destillation von niedrigsiedenden Bestandteilen getrennt. Darauf folgt noch eine vierte Detsillation zur endgültigen Reinigung.

Problematisch an diesem Verfahren ist die Schwefelsäure, die in großen Mengen zugegeben werden muss und in Teilen der Anlage korrosiv wirken kann. Entsprechend müssen diese Teile, wie insbesondere der Phasenserperator oder auch die zweite Derstillationskolonne aus dafür geeigneten Materialien gefertigt sein. Weiterhin schweigt auch die US 7,012,039 über den Umgang mit der gleichsam anfallenden Methacrylsäure oder dem im Produkt verbleibenden restlichen Methanol. Es ist jedoch zu vermuten, dass die erstgenannte in den Destillationsstufen mit abgetrennt wird, während das Methanol nur teilweise mit dem Methacrolein gewonnen und zurückgeführt werden kann, während der Rest wahrscheinlich in der dritten Destillationsstufe verloren geht.

WO 2014/170223 beschreibt ein ähnliches Verfahren wie US 7,012,039. Es besteht nur der Unterschied, dass in der eigentlichen Reaktion mittels Zugabe einer methanolischen Natriumhydroxidlösung in einer Kreislaufführung der pH-Wert eingestellt wird. Dies dient unter anderem dazu, den Katalysator zu schonen. Weiterhin ist die Abtrennung der wässrigen Phase in der Phasenseperation aufgrund des Salzgehalts einfacher. Es führt jedoch auch dazu, dass die entstehende Methacrylsäure als Natriumsalz vorliegt und später mit der wässrigen Phase abgetrennt und verworfen wird. Bei der Variante einer Schwefelsäurezugabe in der Phasenseperation wird die freie Säure zwar wiedergewonnen. Dafür fällt jedoch Natrium(hydrogen)sulfat an, welches bei der Entsorgung zu anderen Problemen führen kann.

Zusammengefasst sind die folgenden Aspekte der Verfahren gemäß Stand der Technik vor allem in der Kombination miteinander verbesserungswürdig:
- Möglichst hohe Ausbeute
- Erhalt der als Methacrylsäure anfallenden Nebenprodukte und Isolierung derselben oder Zurückführung in die oxidative Veresterung
- Möglichst hohen Grad des Recyclings des nicht umgesetzten Methanols
- Reduzierung der zuzugebenden Mengen an Schwefelsäure und Wasser
- Möglichst saubere Entsorgungsströme bzw. Abgase

### Aufgabe

In Anbetracht des Standes der Technik ist es daher Aufgabe der vorliegenden Erfindung, ein technisch verbessertes Verfahren zur oxidativen Veresterung von Methacrolein zur Verfügung zu stellen, das nicht mit den Nachteilen herkömmlicher Verfahren behaftet ist.

Insbesondere war es Aufgabe der vorliegenden Erfindung, eine Verbesserung der Aufarbeitung des Rohproduktes einer oxidativen Veresterung von Methacrolein mit Methanol zu MMA bereitzustellen und damit die Gesamtausbeute eines solchen Prozesses gegenüber dem Stand der Technik zu verbessern.

Darüber hinaus war es Aufgabe, möglichst viele nicht umgesetzte Edukte oder im Prozess gebildete Neben-oder Zwischenprodukte, insbesondere Methanol, Methacrolein, Methacrylsäure und Wasser, in möglichst hohem Maße zu recyclieren und im Prozess zu den Zielprodukten umzusetzen bzw. optional die Methacrylsäure als solche zu isolieren.

Darüber hinaus lag der vorliegenden Erfindung die Aufgabe zugrunde, Methacrylsäure aus dem System unter Einsatz möglichst geringer Mengen zugegebener Säure zu isolieren, bzw. zu recyceln. Dabei soll die lokale Konzentration und der Gesamtverbrauch dieser Säure möglichst gering gehalten werden.

Weiterhin war es Aufgabe der vorliegenden Erfindung, die Aufarbeitung derart zu gestalten, dass möglichst wenige Kontaktstellen in der Anlage mit starken und damit korrosiven Säuren bestehen. Daraus ergab sich wiederum die Aufgabe, dass die Gesamtanlage in Bezug auf den Werkstoff nur im begrenzten Umfang mit einem entsprechenden Säureschutz ausgestattet sein muss.

Insbesondere bestand auch die Aufgabe, ein Verfahren zur Verfügung zu stellen, das mit einem möglichst geringen Entsorgungsaufwand, insbesondere durch ein reduziertes Anfallen organischer Bestandteile und Säuren im Abfallstrom, betrieben werden kann.

Weiterhin soll das Verfahren gegenüber dem Stand der Technik kostengünstig, insbesondere in Bezug auf die bei der Konstruktion der Anlage zu verwendenden Materialien sein.

### Lösung

Gelöst werden die Aufgaben durch ein Verfahren zur Herstellung von MMA , bei dem in einer ersten Reaktionsstufe (A) in einem Reaktor I (1) Methacrolein hergestellt und dieses in einer zweiten Reaktionsstufe (B) in einem Reaktor II (10) oxidativ mit einem Alkohol, bevorzugt mit Methanol zu einem Alkylmethacrylat, bevorzugt entsprechend zu MMA verestert wird, wobei das Verfahren eine neuartige Aufarbeitung (C) des aus dem zweiten Reaktor (10) ausgetragenen Rohprodukts aufweist. Bei dieser Aufarbeitung (C) wird aus einem Gemisch enthaltend Wasser, den Alkohol, mindestens ein Alkalisalz, Methacrylsäure und eine starke Säure in einer Destillationskolonne IV eine niedrigsiedende Fraktion, enthaltend hauptsächlich Alkohol gewonnen. Dieser Alkohol wird im Weiteren zur Herstellung von Alkylmethacrylaten wieder verwendet.

Erfindungsgemäß wird unter einer starken Säure eine derartige Säure verstanden, die stärker ist als Methacrylsäure. Das bedeutet, dass diese Säure unter Normalbedingungen einen geringeren PKS-Wert als Methacrylsäure aufweist. Eine insbesondere bevorzugte anorganische Säure ist dabei Schwefelsäure. Bei den weniger bevorzugten organischen Säuren kann es sich beispielsweise um Methansulfonsäure oder Toluolsulfonsäure handeln. Beispiel für eine weitere geeignete mineralische Säure ist Phosporsäure.

Bevorzugt wird bei dieser Aufarbeitung (C) ein Strom enthaltend Wasser, den Alkohol, mindestens ein Alkalisalz, eine organische und/oder anorganische, bevorzugt anorganische Säure und Methacrylsäure in einer Destillationsstufe (21), im weiteren als Destillationskolonne IV (21) bezeichnet, in eine niedrigsiedende Fraktion (22), enthaltend den Alkohol, eine Seitenstrom-Fraktion (23), enthaltend Wasser und Methacrylsäure und eine Sumpf-Fraktion (24), enthaltend Wasser, die organische und/oder anorganische Säure und deren Alkalisalze getrennt. Neuartig dabei ist insbesondere der getrennt entnommene Seitenstrom (23).

Das Verfahren zur Synthese von MMA, welches die beiden oben aufgeführten Reaktionsstufen (A) und (B) aufweist, kann insbesondere in US 5,969,178, US 7,012,039 und WO 2014/170223 nachgelesen werden. Dabei ist erfindungsgemäß die erste Stufe des Verfahrens zur Synthese des Methacroleins frei wählbar. Das erfindungsgemäße Verfahren ist sowohl auf eine Erststufen-Synthese auf Basis von tert-Butanol oder Isobutylen, als auch auf Basis von Propanal und Formaldhyd anwendbar. Bevorzugt wird die oxidative Veresterung in flüssiger Phase bei einem Druck von 2 bis 100 bar, bevorzugt bei einem Druck im Bereich von 2 bis 50 bar und einer Temperatur im Bereich von 10 bis 200 °C mit einem heterogenen Katalysator durchgeführt. Bei dem heterogenen Katalysator handelt es sich in der Regel um geträgerte goldhaltige Nanopartikel mit einer Teilchengröße kleiner 20 nm, bevorzugt zwischen 0,2 und 20 nm. Die Reaktionsstufe (A) kann eine optionale und weniger bevorzugte Destillationskolonne II zur Abtrennung von Leichtsiedern, wie verbliebenen Propionaldehyd, und/oder von Schwersiedern, wie dimeres Methacrolein, aufweisen.

Bevorzugt handelt es sich bei dieser Destillationsstufe um eine in den folgenden Aufbau integrierte Destillationskolonne IV (21). Das Rohprodukt der oxidativen Veresterung wird zunächst in einer Destillationskolonne III (15) von Methacrolein und teilweise von dem Alkohol, wie beispielsweise Methanol befreit. Dabei wird ein Strom, enthaltend ein Alkylmethacrylat, bevorzugt MMA, Wasser, ein Alkalimethacrylat und/oder Methacrylsäure und Methanol, erhalten. Dieser Strom wird anschließend in einer Extraktion (20) und/oder in einer, ohne eine Extraktion vorliegende oder dieser vorgeschalteten Phasentrennung (19) in eine leichte Phase, enthaltend ein Alkylmethacrylat, bevorzugt MMA, und Methacrylsäure und eine schwere Phase, enthaltend Wasser, Methanol und das Alkalimethacrylat getrennt. Danach wird diese schwere Phase in die Destillationskolonne IV (21) geleitet und der dort entnommene Seitenstrom der Destillationskolonne IV (21) in die Extraktion (20) zurückgeführt. Während dieser Prozessführung erfolgt dabei auch die Zugabe der starken Säure (beispielsweise als (17)), beispielsweise vor der Zuleitung in die Phasentrennung (19) oder in die Extraktion (20).

Bevorzugt wird dabei zwischen der Destillationskolonne III (15) und der Extraktion (20), in die Extraktion (20) selbst, in die aus der Extraktion (20) entnommene schwere Phase und/oder in die Destillationskolonne IV (21) die starke Säure (z.B.17) zugeleitet. Insbesondere bevorzugt wird der das Alkylmethacrylat enthaltene Strom aus Destillationskolonne III mit der starken Säure versetzt.

Für den Fall, dass die starke Säure zumindest teilweise schon vor der Extraktion (20) zugeleitet wird, enthält der Strom aus Destillationskolonne III (15) auch bereits Anteile dieser Säure, sowie entsprechende Alkalisalze der Säure. In diesem Fall liegt die Methacrylsäure als freie Säure vor.

Ein erfindungsgemäß essentielles Merkmal der vorliegenden Erfindung ist die Destillationskolonne IV (21), besonders bevorzugt aufweisend die Seitenstromentnahme. Das bedeutet insbesondere, dass im folgenden beschriebene Aspekte, die nicht spezifisch auf die bevorzugt Ausführungsform mit einer Destillationskolonne III (15) und eine Extraktion (20) gerichtet sind, nicht auf diese Ausführungsformen beschränkt sind. Die Destillationskolonne IV (21) kann außerdem auch als Desorptionskolonne bezeichnet werden.

Diese bevorzugte Aufarbeitung ist darüber hinaus bevorzugt dadurch gekennzeichnet, dass der Zulauf der Extraktion, die Extraktion selbst, die aus der Extraktion entnommene schwere Phase und/oder die Destillationskolonne IV (21) mit der starken, bevorzugt ausschließlich anorganischen Säure versetzt wird. Insbesondere bevorzugt wird die starke Säure direkt vor der Destillationskolonne IV der schweren Phase der Extraktion zugeleitet.

In einer weiteren optionalen, vorteilhaften Variante der Erfindung wird der das Alkylmethacrylat enthaltene Strom aus Destillationskolonne III (15) in einen Phasentrenner (19) geleitet, aus dem eine organische Phase in die Extraktion (20) und eine wässrige Phase in die Destillationskolonne IV (21) weitergeleitet wird.

In einer weiteren optionalen, vorteilhaften Variante der Erfindung wird der das Alkylmethacrylat enthaltene Strom aus Destillationskolonne III (15) in einen Mischer (16) geleitet, in den die starke Säure und optional Wasser zugeführt und mit dem Alkylmethacrylat enthaltenen Strom gemischt werden, und dass diese Mischung anschließend in den Phasentrenner (19) oder direkt in die Extraktion (20) geleitet wird. Bei der Versetzung mit der starken Säure erfolgt auch eine Acetalspaltung. Die Acetale können als Verunreinigung in der Mischung vorliegen. Eine solche Acetalspaltung ist beispielsweise in JP11-302224A beschrieben.

Ein großer Vorteil dieses Vorgehens ist es, dass das Alkalimethacrylat in der Destillationskolonne IV (21) unter Vorliegen der stärkeren, insbesondere dabei anorganischen Säure nahezu vollständig zu Methacrylsäure umgesetzt wird und über den Seitenstrom (23) zurück in die Extraktion (20) geleitet wird. Dort wird diese dann als Bestandteil der niedrigsiedende Fraktion zusammen mit dem MMA aus dem System ausgeschleust und kann isoliert werden. Auf diese Weise erhält man eine insgesamt höhere Ausbeute an gewünschten C₄-Produkten in diesem Verfahren. Andernfalls würde die Methacrylsäure, insbesondere in Form als Alkalisalz, in der wässrigen Phase der Destillationskolonne IV (21) entnommen und z.B. der Entsorgung (24) zugeleitet.

Es ist insbesondere bevorzugt, die anorganische Säure ganz oder zumindest größtenteils direkt vor der Destillationskolonne IV (21) der schweren Phase der Extraktion zuzuleiten. Diese Ausführungsform hat den großen Vorteil, dass nur diese Zuleitung, die Kolonne der Destillationskolonne IV (21) und die Leitung zur Entnahme der Sumpf-Fraktion (24) aus dieser Kolonne, sowie die auf diese folgenden Komponenten aus einem besonders korrosionsbeständigen Material oder mit einer korrosionsbeständigen Beschichtung gefertigt werden müssen. Ein Beispiel für ein solches korrosionsbeständiges Material ist Zirkon. Die Ansprüche, die bei dieser Ausführung an die Extraktion (20) und die Destillationskolonne III (15) gestellt werden, sind dagegen geringer und es kann ein einfacheres Material verwendet werden, was wiederum Investitionskosten spart.

Auch bevorzugt wird dem jeweiligen Strom, insbesondere nach der Destillationskolonne III (15) oder in die Extraktion (20), nur so viel der starken Säure zugegeben, dass der pH-Wert in der Extraktion immer ≥ 3 ist.

Neben dem beschriebenen bevorzugten Aufbau sind erfindungsgemäß auch andere Konstruktionen, die erfindungsgemäße Destillationskolonne IV (21) mit der bevorzugt vorliegenden Seitenstromentnahme aufweisend, denkbar. So ist eine Anlage denkbar, bei der auf die Destillationskolonne III (15) verzichtet wird. Auch wäre anstelle der Extraktion (20) eine einfache Phasentrennung, z.B. in Form einer Zentrifuge denkbar. Auch können mehrere Destillationsstufen, Phasentrtenner und/oder Zentrifugen zur Entnahme der einzelnen Fraktionen in Reihe geschaltet sein.

Dennoch ist es in einer besonderen Ausführungsform trotzdem möglich, dem jeweiligen Strom nach der Destillationskolonne III (15) oder in der Extraktion (20) kleine Mengen der starken Säure zuzuleiten. Hierbei ist dann, wenn man weiterhin entsprechende Materialien für die Destillationskolonne III (15) und die Extraktion (20) verwenden möchte, nötig, dass die zugeleitete Menge der anorganischen Säure derart gewählt wird, dass der entsprechende Strom mit einem pH-Wert von nicht unter 3 eingestellt wird. Vorteil dieser Variante ist es, dass bereits vor oder bei der Zuleitung des Roh-MMA in die Extraktion (20) ein Teil des Alkalimethacrylats zur freien Säure umgesetzt wird und noch vor der Leitung in die Destillationskolonne IV (21) dem System entnommen werden kann.

Insbesondere handelt es sich bei dem erwähnten Alkalisalz um Natriumsulfat, bei dem Alkalimethacrylat um Natriummethacrylat und bei der anorganischen Säure um Schwefelsäure. Es ist aber auch denkbar, andere Alkalimetalle, wie zum Beispiel Kalium einzusetzen. Auch sehr bevorzugt ist es, dass alle aufgeführten Stufen und damit das jeweils gesamte Verfahren unabhängig von der genauen Ausgestaltung kontinuierlich durchgeführt werden.

Folgend werden Verfahren zur weiteren Verarbeitung die der beschriebenen Aufarbeitung entnommenen Ströme beschrieben:
Die leichte Phase, die in der bevorzugten Ausführungsform der Extraktion entnommen wird, kann beispielsweise über mindestens zwei, bevorzugt mindestens drei weitere Destillationsstufen aufgearbeitet werden. Dazu werden dem Roh-MMA zunächst schwersiedende Bestandteile in einer Destillationskolonne V (25) und anschließend leichtsiedende Bestandteile in einer Destillationskolonne VI (26) entnommen. Darüber hinaus kann optional danach noch eine Endreinigung in einer Destillationskolonne VII (27), z.B. zur erneuten Entfernung weiterer schwersiedender Bestandteile erfolgen. Das aufgereinigte Alkylmethacrylat, insbesondere MMA (28) fällt somit z.B. als Kopfstrom einer dritten Destillationsstufe an, während die Methacrylsäure als Sumpf der ersten (29) und/oder dritten Destillationskolonne isoliert und optional einer weiteren Destillationsstufe zugeführt werden kann. Alternativ können auch eine, mehrere oder alle dieser Destillationsstufen, insbesondere zur Reinigung der Methacrylsäure, durch eine Kristallistion ersetzt werden. Unabhängig von dem Vorgehen werden eine überwiegend aus Methacrylsäure (29) und eine überwiegend aus MMA (28) bestehende Fraktion gewonnen.

Die überwiegend aus Methacrylsäure bestehende Fraktion (29) kann im weiteren mit einem Alkohol zu einem Alkylmethacrylat verestert werden. Insbesondere kann die Methacrylsäure mit Methanol zu MMA verestert werden. Bevorzugt erfolgt dies in einem separaten Reaktor (30) und nicht durch zurückleiten in den Reaktor II (10). Dies begründet sich daraus, dass entweder ein zu hoher Säuregehalt in dem Reaktor zur oxidativen Veresterung den dort verwendeten Katalysator schädigen würde oder dort - insbesondere - mittels Zugabe eines Alkalihydroxids, z.B. Natriumhydroxids, ein pH-Wert von ca. 7 eingestellt wird, wodurch die Methacrylsäure wieder zu einem Alkalisalz umgesetzt und eine Veresterung dadurch unterdrückt würde.

Die niedrigsiedende Fraktionen der Destillationskolonne III (15), enthaltend das im Roh-Produkt der oxidativen Veresterung verbliebene Methacrolein und einen Anteil des nicht umgesetzten Methanols, dagegen kann bevorzugt zurück in den Reaktor zur oxidativen Veresterung (10) in der zweiten Reaktionsstufe geführt werden. Das gleiche gilt auch für die niedrigsiedende Fraktion (22) der Destillationskolonne IV (21), enthaltend überwiegend das übrige Methanol. Auch dieses wird bevorzugt zurück in den Reaktor der zweiten Reaktionsstufe (10) geleitet.

Insbesondere enthält die niedrigsiedende Fraktion der Destillationskolonne IV mehr als 60 Gew%, bevorzugt mehr als 65 Gew% Alkohol und weniger als 20 Gew%, bevorzugt weniger als 10 Gew% Wasser. Die Sumpf-Fraktion enthält insbesondere mehr als 60 Gew%, bevorzugt mehr als 80 Gew% Wasser und die bevorzugt vorliegende Seitenstrom-Fraktion der Destillationskolonne IV enthält insbesondere mehr als 80 Gew%, bevorzugt mehr als 95 Gew% Wasser und weniger als 5 Gew%, bevorzugt weniger als 1 Gew% Alkohol.

Die schwere Phase oder gleich bedeutend Sumpf-Fraktion (24) der zweiten Destillationskolonne IV (21) kann schließlich einer Entsorgung (24), z.B. einer biologischen Aufarbeitung oder einer oxidativen Verbrennung zugeführt werden.

Insbesondere bevorzugt ist es, wenn der Seitenstrom (23) der Kolonne der Destillationskolonne IV (21) an einer Stelle dieser Kolonne entnommen wird, in der der Alkoholgehalt, bzw. bevorzugt der Methanolgehalt kleiner 1 Gew% ist. Bevorzugt weist die Kolonne dabei Siedeböden auf, so dass der Seitenstrom (23) flüssig entnommen wird und nicht zusätzlich kondensiert werden muss. Bei einer flüssigen Entnahme enthält der Seitenstrom (23) jedoch unter Umständen einen Anteil der starken Säure, insbesondere Schwefelsäure, die damit zurück in die Extraktion (20) geführt würde. Für den Fall, dass diese Mengen nur gering sind und damit in der Extraktion ein pH-Wert oberhalb von 3 erzielt wird, stellt dies kein größeres Problem dar. Sollte der Seitenstrom (23) jedoch derart entnommen werden, dass der Säuregehalt größer ist, müsste auch die Extraktion (20) aus einem entsprechenden korrosionsbeständigen Material konstruiert werden.

Alternativ und besonders bevorzugt weist die Destillationskolonne IV (21) im unteren Teil eine Trennwand auf. In dieser Ausführungsform wird der Kolonnenfeed in die Destillationskolonne IV (21) am oberen Ende der Trennwand zugeführt und der Seitenabzug an einer Stelle auf der anderen Seite der Trennwand derart platziert, dass an der die Konzentration der starken Säure kleiner 0,1 Gew% ist und andererseits der Alkoholgehalt kleiner 1 Gew% ist. In diesem Fall kann der Seitenstrom bevorzugt am oberen Rand dieser Seite der Trennwand flüssig entnommen werden. Diese Variante hat zudem die großen Vorteile, dass insbesondere kaum Schwefelverbindungen im Wasser der Entnahmeseite der Trennwand vorliegen, und dass es auf der anderen Seite der Trennwand zu einer längeren Verweilzeit für die Umsetzung des Alkylmethacrylats mit der Schwefelsäure kommt. Weiterhin kann dann auch die Leitung zur Überführung des Seitenstroms zurück in die Extraktion (20) aus weniger korrosionsbeständigem Material gebaut werden. Destillationskolonnen mit einer Trennwand können beispielsweise in N. Asprion, G. Kaibel, Chem. Eng. and Process., 49 (2010), 139-146 oder in I. Dejanovic et al., Chem. Eng. and Process., 49 (2010), 559-580 nachgelesen werden.

Besonders bevorzugt wird zusätzlich ein Wärmetauscher in die Anlage eingebaut, mittels dem die Seitenstromfraktion (23) der Destillationskolonne IV (21) vor der Einleitung in die Extraktion (20) abgekühlt und gleichzeitig die schwere Phase der Extraktion (20) vor der Einleitung in die Destillationskolonne IV (21) erwärmt wird.

Unabhängig von den konkreten Ausgestaltungen des erfindungsgemäßen Verfahrens hat dieses nur im jeweiligen Grad unterschiedlich den großen Vorteil gegenüber den bekannten Verfahren des Standes der Technik, dass insbesondere die lokalen Konzentration der starken Säuren, wie insbesondere Schwefelsäure, lokal gesteuert werden kann und insbesondere in der Destillationsstufe III (15), dem optionalen Phasentrenner (19) und der Extraktion (20), sowie den dazwischen befindlichen Leitungen und optionalen weiteren Bauteilen gering gehalten werden kann. Dies hat nicht nur den Vorteil, dass weniger korrosionsbeständige Materialien verwendet werden können, sondern insbesondere auch, dass das Alkylmethacrylat, die dazu eingesetzten und verbliebenen Rohstoffe, sowie die gebildeten Nebenprodukte nicht oder nur in geringerem Maße dieser Säure ausgesetzt werden. Dies hätte die Bildung weiterer Nebenprodukte, einhergehend mit einer verminderten Ausbeute und einer eventuellen Färbung des Endproduktes zur Folge. Weiterhin ergibt sich parallel dazu der Vorteil, dass insgesamt weniger Säure eingesetzt werden muss.

Ein weiterer sehr großer Vorteil der vorliegenden Erfindung ist es, dass die Sumpf-Fraktion (24) weniger Säure, Produkt, Edukte und Nebenprodukte enthält. Durch die Recyclierung der abgetrennten Produkte Alkylmethacrylat und Methacrylsäure wird die C₄-Ausbeute des Gesamtverfahrens merklich gesteigert. Durch die Abtrennung der Edukte, insbesondere des Alkohols und die optionale Recyclierung in Reaktor II (10) erfolgt eine weitere Effizienzsteigerung des Verfahrens. Insbesondere aber ist die wässrige Sumpf-Fraktion (24) mit allen diesen aufgeführten Bestandteilen weniger belastet, was zum einen die Entsorgung der Fraktion vereinfacht und weiterhin die Umwelt auch bei natürlich sachgerechter Entsorgung weniger belastet. Insbesondere kann dieser Sumpfstrom (24) aus der Destillationskolonne IV (21) einer weiteren Trennstufe zur Rückgewinnung von Wasser und Methacrylsäure und zum Aufkonzentrieren des Salz-haltigen Abfallstrom zugeführt werden. Bei einer solchen Trennstufe kann es sich beispielsweise um eine Stripping-Kolonne, eine Membrantrennstufe oder eine Kombination aus verschiedener solcher Elemente handeln. Eine Stripping-Kolonne wird dabei deutlich heißer und damit schneller als die Destillationskolonne IV (21) betrieben. Eine solche weitere Aufarbeitung dient insbesondere zur Rückgewinnung und Recyclierung von Methacrylsäure und Wasser aus dem dann aufkonzentrierten, salzhaltigen Abfallstrom.

In einer besonders bevorzugten Variante wird der Sumpfstrom derart aufgearbeitet, dass die Salzkonzentration nach dem Aufkonzentrieren nah zu der Konzentration einer gesättigten Lösung ist. Damit wird vorteilhafter weise zugleich die Methacrylsäure-Menge im Abfallstrom minimiert. Weiterhin können auch durch eine entsprechende Schaltung, zum Beispiel verschiedener Membrantrennstufen, zwei Abfallströme erzeugt werden. Dabei ist einer dieser beiden bzgl. des Salz- und Nebenproduktgehalts hochkonzentriert, während der andere überwiegend aus Wasser besteht. Dies hat den Vorteil, dass der gering-konzentrierte zweite Abfallstrom beispielsweise einer biologischen Entsorgung zugeführt werden kann. Der hochkonzentrierte Strom wird in der Regel einem Thermal Oxidizer zugeführt.

### Bezugszeichenliste

### (A) Synthese und Isolierung des Methacroleins

### ((1) - (8), (33)-(35))

(1) Reaktor I zur Methacrolein-Synthese
(2) Zulauf Formaldehyd
(3) Zulauf Propionaldehyd
(4) Zulauf Base I
(5) Zulauf Säure I
(6) Destillationskolonne I, Katalysatorabtrennung
(7) Optionale Rückführung Katalysatorfraktion in Reaktor I
(8) Phasentrennung MAL, Methacrolein-Isolierung
(33) Rückführung MAL-haltiger, wässriger Phase in Destillationskolonne I (6)
(34) Abfallstrom der Methacrolein-Synthese
(35) Strom zur Überführung des Methacroleins aus MAL-Synthese (A) in Oxidative Veresterung (B); optional, jedoch nicht bevorzugt aufweisend eine Destillationskolonne II

### (B) Oxidative Veresterung des Methacroleins zu einem Alkylmethacrylat und Recyclierung des Methacroleins

### ((9) - (15))

(9) Methacroleinstrom-Zulauf in Reaktor II
(10) Reaktor II zur oxidativen Veresterung des Methacroleins
(11) Alkohol-Zulauf (i.d.R. Methanol-Zulauf)
(12) Sauerstoff- bzw. Luftzuleitung
(13) Zulauf Base II
(14) Recyclingstrom, enthaltend Methacrolein und Alkohol
(15) Destillationskolonne III zur Trennung von Methacrolein und teilweise Alkohol von Roh-Aklymethacrylat

### (C) Erfindungsgemäß bevorzugte Trennung des Reaktoraustrags aus Reaktor II

### ((16) - (24))

(16) Optionaler Mischer
(17) Zulauf Säure II
(18) Optionaler Zulauf Wasser
(19) Phasentrennung
(20) Extraktion
(21) Erfindungsgemäße Destillationsstufe (Destillationskolonne IV)
(22) Niedrigsiedende Fraktion, enthaltend Alkohol zur Rückführung in Reaktor II
(23) Seitenstromfraktion, enthaltend Wasser und Methacrylsäure, zur Rückführung in die Extraktionsstufe (20), Mischer (16) oder Phasentrennung (19).
(24) Sumpf-Fraktion, enthaltend Wasser, Säure II und deren Alkalisalze, zur Entsorgung oder weiterer Aufarbeitung

### (D) Bevorzugte Aufarbeitung des rohen Alkylmethacrylats (z.B. Roh-MMA)

### ((25) - (32))

(25) Destillationskolonne V zur Abtrennung von Schwersiedern
(26) Destillationskolonne VI zur Abtrennung von Leichtsiedern
(27) Destillationskolonne VII zur Endreinigung des Alkylmethacrylats
(28) Alkylmethacrylat-Produktstrom
(29) Methacrylsäure-haltiger Strom aus Destillationskolonne V (25)
(30) Optionale Veresterung der Methacrylsäure zu Alkylmethacrylat, einschließlich Trennung einer Alkylmethacrylat-haltigen Phase von einer Abfall-Phase
(31) Optionale Isolierung reiner Methacrylsäure als zweites Produkt
(32) Abfallstrom der Veresterung (30)

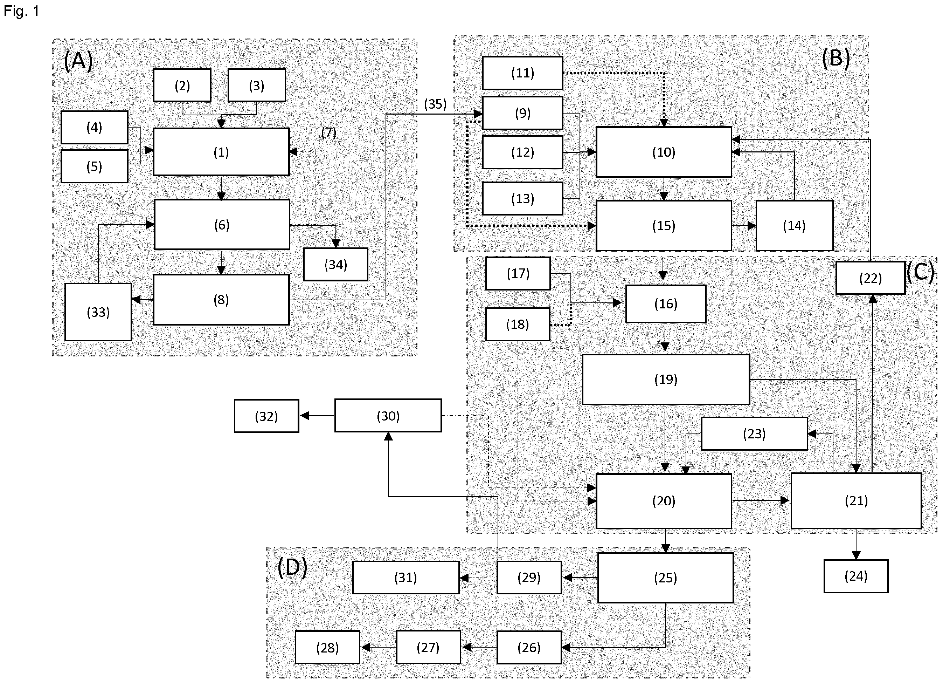

## Patentansprüche

1. Verfahren zur Herstellung von MMA, bei dem in einer ersten Reaktionsstufe in einem Reaktor I Methacrolein hergestellt und dieses in einer zweiten Reaktionsstufe in einem Reaktor II oxidativ mit einem Alkohol zu einem Alkylmethacrylat verestert wird, **dadurch gekennzeichnet, dass** aus einem Gemisch enthaltend Wasser, den Alkohol, mindestens ein Alkalisalz, Methacrylsäure und eine starke Säure in einer Destillationskolonne IV eine niedrigsiedende Fraktion, enthaltend hauptsächlich Alkohol gewonnen wird und dieser Alkohol zur Herstellung von Alkylmethacrylaten wieder verwendet wird.

2. Verfahren gemäß Anspruch 1, **dadurch gekennzeichnet, dass** das Roh-Alkylmethacrylat der oxidativen Veresterung in einer Destillationskolonne III von Methacrolein und teilweise von dem Alkohol befreit wird, wobei ein Strom, enthaltend Alkylmethacrylat, Wasser, ein Alkalimethacrylat und/oder Methacrylsäure und Alkohol, erhalten wird, mit einer starken Säure versetzt und in einer Phasentrennung und/oder einer Extraktion in eine leichte Phase, enthaltend Alkylmethacrylat und Methacrylsäure, und eine schwere Phase, enthaltend Wasser, den Alkohol mindestens ein Alkalisalz und Methacrylsäure, welche direkt oder indirekt in die Destillationskolonne IV geleitet wird, getrennt wird

3. Verfahren gemäß Anspruch 1 oder 2, **dadurch gekennzeichnet, dass** ein Gemisch enthaltend Wasser, den Alkohol, mindestens ein Alkalisalz, eine starke Säure und Methacrylsäure in einer Destillationskolonne IV in eine niedrigsiedende Fraktion, enthaltend den Alkohol, eine Seitenstrom-Fraktion, enthaltend Wasser und Methacrylsäure und eine Sumpf-Fraktion, enthaltend Wasser, die starke Säure und deren Alkalisalze getrennt wird.

4. Verfahren gemäß mindestens einem der Ansprüche 1 bis 3, **dadurch gekennzeichnet, dass** der das Alkylmethacrylat enthaltene Strom aus Destillationskolonne III mit der starken Säure versetzt wird und in eine Phasentrennung geleitet wird, aus der eine organische Phase in die Extraktion und eine wässrige Phase in die Destillationskolonne IV geleitet wird.

5. Verfahren gemäß mindestens einem der Ansprüche 2 bis 4, **dadurch gekennzeichnet, dass** der das Alkylmethacrylat enthaltene Strom aus Destillationskolonne III in einen Mischer geleitet wird, in den die starke Säure und optional Wasser zugeführt und mit dem Alkylmethacrylat enthaltenen Strom gemischt werden, und dass diese Mischung anschließend in den Phasentrenner und/oder in die Extraktion geleitet wird.

6. Verfahren gemäß mindestens einem der Ansprüche 2 bis 5, **dadurch gekennzeichnet, dass** die starke Säure direkt vor der Destillationskolonne IV der schweren Phase der Extraktion zugeleitet wird.

7. Verfahren gemäß mindestens einem der Ansprüche 1 bis 6, **dadurch gekennzeichnet, dass** es sich bei dem Alkalisalz um Natriumsulfat, bei dem Alkalimethacrylat um Natriummethacrylat, bei der anorganischen Säure um Schwefelsäure bei dem Alkohol um Methanol und bei dem Alkylmethacrylat um MMA handelt.

8. Verfahren gemäß mindestens einem der Ansprüche 2 bis 7, **dadurch gekennzeichnet, dass** die leichte Phase der Extraktion mindestens zwei weiteren Destillationsstufen und/oder einer Kristallisation zugeleitet wird, und dass dabei eine überwiegend aus Methacrylsäure und eine überwiegend aus MMA bestehende Fraktion gewonnen werden.

9. Verfahren gemäß Anspruch 8, **dadurch gekennzeichnet, dass** die überwiegend aus Methacrylsäure bestehende Fraktion mit einem Alkohol zu einem Alkylmethacrylat verestert wird.

10. Verfahren gemäß mindestens einem der Ansprüche 2 bis 9, **dadurch gekennzeichnet, dass** dem jeweiligen Strom nach der Destillationskolonne III oder in die Extraktion nur so viel der starken Säure zugegeben wird, dass der pH-Wert in der Extraktion immer ≥ 3 ist.

11. Verfahren gemäß mindestens einem der Ansprüche 2 bis 10, **dadurch gekennzeichnet, dass** die niedrigsiedenden Fraktionen der Destillationskolonne III und/oder der Destillationskolonne IV in den Reaktor II geleitet werden.

12. Verfahren gemäß mindestens einem der Ansprüche 1 bis 11, **dadurch gekennzeichnet, dass** die niedrigsiedende Fraktion der Destillationskolonne IV mehr als 60 Gew% Alkohol und weniger als 20 Gew% Wasser enthält, die Sumpf-Fraktion mehr als 60 Gew% Wasser enthält und die Seitenstrom-Fraktion der Destillationskolonne IV mehr als 80 Gew% Wasser und weniger als 5 Gew% Alkohol enthält.

13. Verfahren gemäß mindestens einem der Ansprüche 1 bis 12, **dadurch gekennzeichnet, dass** die Destillationskolonne IV, im unteren Teil eine Trennwand aufweist, dass der Kolonnenfeed am oberen Ende der Trennwand zugeführt wird, und dass der Seitenabzug an einer Stelle auf der anderen Seite der Trennwand, an der die Konzentration der starken Säure kleiner 0,1 Gew% ist und andererseits der Alkoholgehalt kleiner 1 Gew% ist, flüssig entnommen wird.

14. Verfahren gemäß mindestens einem der Ansprüche 1 bis 13, **dadurch gekennzeichnet, dass** der Sumpfstrom aus der Destillationskolonne IV einer weiteren Trennstufe zur Rückgewinnung von Wasser und Methacrylsäure und zum Aufkonzentrieren des Salz-haltigen Abfallstroms zugeführt wird.

## Claims

1. Process for producing MMA where in a first reaction stage in a reactor I methacrolein is produced and in a second reaction stage in a reactor II said methacrolein is oxidatively esterified with an alcohol to afford an alkyl methacrylate, **characterized in that** a low-boiling fraction comprising mainly alcohol is obtained from a mixture comprising water, the alcohol, at least one alkali metal salt, methacrylic acid and a strong acid in a distillation column IV, and this alcohol is reused for producing alkyl methacrylates.

2. Process according to Claim 1, **characterized in that** the crude alkyl methacrylate from the oxidative esterification is freed of methacrolein and, partially, of the alcohol in a distillation column III, wherein a stream comprising alkyl methacrylate, water, an alkali metal methacrylate and/or methacrylic acid and alcohol is obtained, admixed with a strong acid and separated in a phase separation and/or an extraction into a light phase comprising alkyl methacrylate and methacrylic acid and a heavy phase comprising water, the alcohol, at least one alkali metal salt and methacrylic acid which is directly or indirectly passed into the distillation column IV.

3. Process according to either of Claims 1 and 2, **characterized in that** a mixture comprising water, the alcohol, at least one alkali metal salt, a strong acid and methacrylic acid is separated in a distillation column IV into a low-boiling fraction comprising the alcohol, a sidestream fraction comprising water and methacrylic acid and a bottoms fraction comprising water, the strong acid and the alkali metal salts thereof.

4. Process according to at least one of Claims 1 to 3, **characterized in that** the stream from distillation column III comprising the alkyl methacrylate is admixed with the strong acid and passed into a phase separation from which an organic phase is passed into the extraction and an aqueous phase is passed into the distillation column IV.

5. Process according to at least one of Claims 2 to 4, **characterized in that** the stream from distillation column III comprising the alkyl methacrylate is passed into a mixer into which the strong acid and optionally water are sent and mixed with the alkyl-methacrylate-comprising stream and that this mixture is subsequently passed into the phase separator and/or into the extraction.

6. Process according to at least one of Claims 2 to 5, **characterized in that** the feeding of the strong acid into the heavy phase from the extraction is effected directly upstream of the distillation column IV.

7. Process according to at least one of Claims 1 to 6, **characterized in that** the alkali metal salt is sodium sulfate, the alkali metal methacrylate is sodium methacrylate, the inorganic acid is sulfuric acid, the alcohol is methanol and the alkyl methacrylate is MMA.

8. Process according to at least one of Claims 2 to 7, **characterized in that** the light phase from the extraction is fed into at least two further distillation stages and/or a crystallization and that a fraction composed predominantly of methacrylic acid and a fraction composed predominantly of MMA are thus obtained.

9. Process according to Claim 8, **characterized in that** the fraction composed predominantly of methacrylic acid is esterified with an alcohol to afford an alkyl methacrylate.

10. Process according to at least one of Claims 2 to 9, **characterized in that** the strong acid is added to the respective stream downstream of the distillation column III or into the extraction only in an amount sufficient to ensure that the pH in the extraction is always ≥ 3.

11. Process according to at least one of Claims 2 to 10, **characterized in that** the low-boiling fractions from the distillation column III and/or the distillation column IV are passed into the reactor II.

12. Process according to at least one of Claims 1 to 11, **characterized in that** the low-boiling fraction from the distillation column IV comprises more than 60 wt% of alcohol and less than 20 wt% of water, the bottoms fraction comprises more than 60 wt% of water and the sidestream fraction from the distillation column IV comprises more than 80 wt% of water and less than 5 wt% of alcohol.

13. Process according to at least one of Claims 1 to 12, **characterized in that** the lower part of the distillation column IV comprises a dividing wall, that the column feed is supplied at the upper end of the dividing wall and that the side draw is taken off in liquid form at a point on the other side of the dividing wall where the concentration of the strong acid is less than 0.1 wt% while the alcohol content is less than 1 wt%

14. Process according to at least one of Claims 1 to 13, **characterized in that** the bottoms stream from the distillation column IV is sent to a further separating stage to recover water and methacrylic acid and to concentrate the salt-containing waste stream.

## Revendications

1. Procédé de fabrication de MMA, selon lequel, lors d'une première étape de réaction dans un réacteur I, de la méthacroléine est fabriquée, et celle-ci est estérifiée lors d'une deuxième étape de réaction dans un réacteur II par oxydation avec un alcool pour former un méthacrylate d'alkyle, **caractérisé en ce qu'**une fraction de point d'ébullition faible contenant principalement l'alcool est obtenue dans une colonne de distillation IV à partir d'un mélange contenant de l'eau, l'alcool, au moins un sel alcalin, de l'acide méthacrylique et un acide fort, et cet alcool est réutilisé pour la fabrication de méthacrylates d'alkyle.

2. Procédé selon la revendication 1, **caractérisé en ce que** le méthacrylate d'alkyle brut de l'estérification oxydative est débarrassé de la méthacroléine et en partie de l'alcool dans une colonne de distillation III, un courant contenant du méthacrylate d'alkyle, de l'eau, un méthacrylate alcalin et/ou de l'acide méthacrylique et l'alcool étant obtenu, mélangé avec un acide fort et séparé dans une séparation des phases et/ou une extraction en une phase légère, contenant le méthacrylate d'alkyle et l'acide méthacrylique, et une phase lourde, contenant de l'eau, l'alcool, au moins un sel alcalin et l'acide méthacrylique, qui est directement ou indirectement introduite dans la colonne de distillation IV.

3. Procédé selon la revendication 1 ou 2, **caractérisé en ce qu'**un mélange contenant de l'eau, l'alcool, au moins un sel alcalin, un acide fort et de l'acide méthacrylique est séparé dans une colonne de distillation IV en une fraction de point d'ébullition faible, contenant l'alcool, une fraction de courant latéral, contenant de l'eau et de l'acide méthacrylique, et une fraction de fond, contenant de l'eau, l'acide fort et ses sels alcalins.

4. Procédé selon au moins l'une quelconque des revendications 1 à 3, **caractérisé en ce que** le courant contenant le méthacrylate d'alkyle issu de la colonne de distillation III est mélangé avec l'acide fort et introduit dans une séparation des phases, à partir de laquelle une phase organique est introduite dans l'extraction et une phase aqueuse dans la colonne de distillation IV.

5. Procédé selon au moins l'une quelconque des revendications 2 à 4, **caractérisé en ce que** le courant contenant le méthacrylate d'alkyle issu de la colonne de distillation III est introduit dans un mélangeur, dans lequel l'acide fort et éventuellement de l'eau sont introduits et mélangés avec le courant contenant le méthacrylate d'alkyle, et **en ce que** ce mélange est ensuite introduit dans le séparateur de phases et/ou dans l'extraction.

6. Procédé selon au moins l'une quelconque des revendications 2 à 5, **caractérisé en ce que** l'acide fort est introduit dans la phase lourde de l'extraction directement avant la colonne de distillation IV.

7. Procédé selon au moins l'une quelconque des revendications 1 à 6, **caractérisé en ce que** le sel alcalin est le sulfate de sodium, le méthacrylate alcalin est le méthacrylate de sodium, l'acide inorganique est l'acide sulfurique, l'alcool est le méthanol et le méthacrylate d'alkyle est le MMA.

8. Procédé selon au moins l'une quelconque des revendications 2 à 7, **caractérisé en ce que** la phase légère de l'extraction est introduite dans au moins deux étapes de distillation supplémentaires et/ou dans une cristallisation, et **en ce qu'**une fraction essentiellement constituée par de l'acide méthacrylique et une fraction essentiellement constituée par du MMA sont obtenues.

9. Procédé selon la revendication 8, **caractérisé en ce que** la fraction essentiellement constituée par de l'acide méthacrylique est estérifiée avec un alcool en un méthacrylate d'alkyle.

10. Procédé selon au moins l'une quelconque des revendications 2 à 9, **caractérisé en ce qu'**uniquement suffisamment de l'acide fort pour que le pH dans l'extraction soit toujours ≥ 3 est ajouté au courant respectif après la colonne de distillation III ou dans l'extraction.

11. Procédé selon au moins l'une quelconque des revendications 2 à 10, **caractérisé en ce que** les fractions de point d'ébullition faible de la colonne de distillation III et/ou de la colonne de distillation IV sont introduites dans le réacteur II.

12. Procédé selon au moins l'une quelconque des revendications 1 à 11, **caractérisé en ce que** la fraction de point d'ébullition faible de la colonne de distillation IV contient plus de 60 % en poids d'alcool et moins de 20 % en poids d'eau, la fraction de fond contient plus de 60 % en poids d'eau, et la fraction de courant latéral de la colonne de distillation IV contient plus de 80 % en poids d'eau et moins de 5 % en poids d'alcool.

13. Procédé selon au moins l'une quelconque des revendications 1 à 12, **caractérisé en ce que** la colonne de distillation IV comprend une paroi de séparation dans la partie inférieure, **en ce que** l'alimentation de la colonne est introduite à l'extrémité supérieure de la paroi de séparation, et **en ce que** le soutirage latéral est soutiré sous forme liquide à un emplacement sur l'autre côté de la paroi de séparation, auquel la concentration de l'acide fort est inférieure à 0,1 % en poids et d'un autre côté la teneur en alcool est inférieure à 1 % en poids.

14. Procédé selon au moins l'une quelconque des revendications 1 à 13, **caractérisé en ce que** le courant de fond de la colonne de distillation IV est introduit dans une étape de séparation supplémentaire pour la récupération d'eau et d'acide méthacrylique, et pour la concentration du courant résiduaire contenant un sel.
